# EUROPÄISCHE PATENTSCHRIFT

(11) **EP 3 302 373 B1**
(45) Veröffentlichungstag und Bekanntmachung des Hinweises auf die Patenterteilung: **07.08.2024**
(21) Anmeldenummer: 16732459.9
(22) Anmeldetag: 03.06.2016
(51) Int. Cl.: A61F 2/915, A61F 2/24, A61F 2/06, A61F 2/07

(54) **GEFÄSSSTÜTZE**
STENT
SUPPORT VASCULAIRE

(30) Priorität: 03.06.2015 DE 102015108835
(43) Veröffentlichungstag der Anmeldung: 11.04.2018
(73) Patentinhaber: Andratec GmbH, 56075 Koblenz (DE)
(72) Erfinder: NEUSS, Malte, 53121 Bonn (DE); PILLAI, Jay, Johannesburg (ZA); KOHL, Gunter, 56068 Koblenz (DE)
(74) Vertreter: Schneiders & Behrendt Bochum
(86) Internationale Anmeldenummer: PCT/EP2016/062684
(87) Internationale Veröffentlichungsnummer: WO 2016/193449

(56) Entgegenhaltungen:
- EP-A1- 1 958 598
- WO-A2-2014/004746
- DE-A1- 102012 001 996
- DE-U1- 202009 012 562
- US-A1- 2011 046 717
- US-A1- 2013 131 780

## Beschreibung

Die Erfindung betrifft eine radial aufweitbare Gefäßstütze, welche eine Mehrzahl von miteinander flexibel verbundenen mäandrierenden Ringelementen aufweist, welche eine Gefäßstütze mit einem proximalen und einem distalen Ende und einer Längsachse definieren, wobei die Ringelemente entlang der Längsachse der Gefäßstütze nebeneinander angeordnet sind und aneinander grenzende Ringelemente durch Verbindungselemente miteinander verbunden sind.

Die erfindungsgemäßen Gefäßstützen sind auf der ganzen Länge oder in Teilbereichen radial aufweitbare Stents zur Verwendung beim Offenhalten von Blutgefäßen oder sonstigen Organwegen in menschlichen oder tierischen Körpern.

Gefäßstützen werden allgemein zur dauerhaften Stützung der Gefäßwand im Bereich von Stenosen implantiert, als Trägerstruktur von Membranen zum Offenhalten von Gefäßen oder von minimal invasiv implantierbaren Herzklappen. Sie werden auf einen zusammengefalteten Ballon aufgepresst und im Gefäß durch Ballondilatation auf einen bestimmten Durchmesser aufgedehnt.

Da bei Kindern aber der maximal einführbare Kathederdurchmesser von einem Ballonkatheter mit montiertem Stent durch den limitierten Durchmesser der Zugangsgefäßkammer der Femoralarterie oder der Femoralvene, begrenzt ist, kann ein herkömmlicher Stent nur bis auf einen Durchmesser von maximal 6 bis 8 mm aufgedehnt werden. Da aber beispielsweise der Durchmesser einer Lungenarterie im Erwachsenenalter bis zu 30 mm betragen kann, müssten herkömmliche Stents mit relativ zu kleinem Durchmesser im Laufe des Wachstums chirurgisch mit einer Operation an der Herzlungenmaschine entfernt werden. Durch die erfindungsgemäßen Stents mit Sollbruchstellen wird dieser Nachteil vermieden, da sie nach Aufbrechen der Sollbruchstellen mit einem weiteren Ballon beliebig weit aufgedehnt werden können. Dann haben sie zwar kaum noch eine Radialfestigkeit, aber gegebenenfalls kann diese durch die Implantation eines weiteren Stents mit größerem Durchmesser erreicht werden.

Andere redilatierbare Gefäßstützen weisen Nahtverbindungen zwischen zickzackförmigen Drahtgerüsten auf, wie beispielsweise gemäß DE 101 03 000, die sich nach einer gewissen Zeit auflösen und damit ein weiteres Gefäßwachstum erlauben. Nachteilig ist ein Verschieben der zusammengenähten Teilsegmente beim Crimpen auf einen Ballon und eine geringe Radialkraft.

Gemäß US 5, 591, 223 A wird eine reexpandierbare Endoprothese aus einem zunächst aufgerollten, steifen rohrförmigen Drahtgeflecht mit einem Längsschlitz gebildet. Der Längsschlitz wird ebenfalls durch resorbierbare Verbindungsfäden zusammengehalten. Durch Auflösen dieser Verbindungsfäden mit der Zeit kann diese Prothese nur im Bereich des einen Schlitzes aufgedehnt werden. Für den Einsatz bei kurvigem Gefäßverlauf wie bei Kindern und bei herznahen Gefäßen ist diese Endoprothese wegen ihrer mangelnden Flexibilität nicht geeignet.

In der WO 2011/032526 A1 ist eine radial aufweitbare Gefäßstütze mit Dehnelementen beschrieben, die eine weitere Aufdehnung in einem großen Durchmesserbereich bei Erhalt der Radialkraft erlauben. Hier ist nachteilig, dass nach zirkulärer Aufdehnung und Bruch der Dehnelemente diese korkenzieherartig die Wand des Blutgefäßes perforieren können.

Aus der US 5, 591, 223 A geht eine reexpandierbare Gefäßstütze hervor, die ein Ringsegment mit einer Wachstumsfuge aufweist. Die Wachstumsfuge wird durch Verbindungsstreifen überbrückt, die aus absorbierbarer Wundnaht bestehen können und zerreißen, wenn eine definierte Zugkraft ausgeübt wird. Die Verbindungsstreifen sind durch Schweißen, Löten oder Kleben am Ringsegment befestigt.

Die EP 1 958 598 A1 beschreibt eine Stützprothese mit Sollbruchstellen gemäß Oberbegriff des Anspruchs 1.

Aufgabe der vorliegenden Erfindung ist es, eine radial aufweitbare Gefäßstütze zu schaffen, die während ihrer Aufweitung keine oder nur eine geringe Verkürzung erfährt, bei besserer Kurvengängigkeit weniger leicht knickt, eine ausreichende Radialfestigkeit aufweist und gleichzeitig durch Aufweiten zumindest von Teilstücken erneut dilatiert werden kann.

Diese Aufgabe wird durch eine radial expandierbare Gefäßstütze mit den Merkmalen von Patentanspruch 1 gelöst.

Die erfindungsgemäßen Gefäßstützen bestehen aus nebeneinander angeordneten Ringelementen, die mäandrierend oder zickzackförmig verlaufen, sowie Verbindungselementen, die nebeneinander liegende Ringelemente miteinander verbinden. Zumindest einige Verbindungselemente weisen Sollbruchstellen auf, die insbesondere parallel zur Längsachse der Gefäßstütze verlaufen, aber auch quer dazu verlaufen können. Ein Verbindungselement kann dabei sowohl längs als auch quer verlaufende Sollbruchstellen aufweisen. Schräg verlaufende Sollbruchstellen sind ebenfalls möglich. Insbesondere kommen aber längsverlaufende Sollbruchstellen infrage, die ein partielles oder vollständiges Öffnen des Stents bei einer Nachdilatation ermöglichen. Dazu erstrecken sich längs verlaufende Sollbruchstellen auch auf und über die Stege der Ringelemente, die durch die jeweiligen Verbindungselemente miteinander verbunden sind.

Insgesamt besteht die erfindungsgemäße flexible radiale reexpandierbare Gefäßstütze aus einem gitterförmigen Stentgerüst mit einer mäandrierenden Ringstruktur von geringer Breite mit mehreren kreisförmig, linear, quer oder spiralig darauf angeordneten Sollbruchstellen in Form von Perforationslinien oder Einkerbungen. Bei der Herstellung und im ersten Implantationszustand bleiben diese Sollbruchstellen geschlossen und miteinander fest verbunden, können aber zu einem späteren Zeitpunkt durch eine weitere Ballondilatation in beliebiger Weise aufgebrochen werden.

Durch das Aufbrechen der Verbindungselemente insgesamt oder in einzelnen Teilbereichen der Gefäßstütze zu einem späteren Zeitpunkt wird ein weiteres Wachstum des Gefäßes ermöglicht, gegebenenfalls auch durch eine weitere Ballondilatation oder die Implantation einer weiteren Gefäßstütze mit größerem Durchmesser in den Bereichen der bestehenden Gefäßstütze.

Durch das seitliche Aufbrechen der Sollbruchstellen einer Gefäßstütze, beispielsweise im Bereich einer durch Markierungen besonders gekennzeichneten Zone mit Sollbruchstellen in den Verbindungselementen, kann durch lokale Aufdehnung in vorteilhafter Weise ein verbesserter Seitenastzugang geschaffen werden.

Der verbesserte Seitenastzugang kann erfindungsgemäß sowohl durch Aufbrechen von Sollbruchstellen in Teilbereichen der Gefäßstütze als auch durch Anordnung von verlängerten Stentstreben oder Weglassen von Stentstreben erreicht werden.

Ist die Gefäßstütze zusätzlich, etwa auf der Zirkumferenz, ganz oder in Teilbereichen mit einer biologischen oder synthetischen Membran gecovert, so kann deren Befestigung auf der Gefäßstütze durch Vernähen, Verkleben, aber auch mit speziellen Klemm- und Clipelementen erfolgen. Diese eignen sich auch zur Befestigung von biologischem oder synthetischem Gewebe, Membranen oder Folien und für mit Laser geschnittenen Schirmsystemen zum Verschließen von Herzwanddefekten. Gleiches gilt für implantierbare Herzklappen.

Ist die Gefäßstütze mit einer Membran gecovert, so ist es besonders vorteilhaft, die beiden jeweils äußeren Ringelemente nicht zu covern, damit es beim Aufdehnen der Gefäßstütze an den Enden nicht zu einem Membranprolabs kommt.

Die erfindungsgemäßen Gefäßstützen weisen eine Mehrzahl von Verbindungselementen mit Sollbruchfunktion auf. Diese Sollbruchfunktionen können beispielsweise auf die Verbindungselemente von nur zwei benachbarten Ringelementen begrenzt sein, um beispielsweise eine Auftrennung oder Aufklappung der Gefäßstütze zu erreichen, sie können aber auch zwischen einer Mehrzahl von Ringelementen angeordnet sein, um beispielsweise einen Bereich zu definieren und auf diese Art und Weise Fenestrierungen zu ermöglichen. Eine weitere Variante ist die Anordnung der Sollbruchstellen über die Länge der Gefäßstütze, so dass diese insgesamt vollständig oder partiell aufgetrennt werden kann. Dabei können die Sollbruchstellen parallel zur Längsachse der Gefäßstütze angeordnet sein, aber auch einer Helix-Linie, die sich um die Gefäßstütze erstreckt, folgen.

Von besonderem Interesse sind Sollbruchstellen, die auf allen Verbindungselementen in einem Teilbereich des Stents angeordnet sind und damit die Öffnung eines Fensters ermöglichen, etwa im Bereich eines abgehenden Blutgefäßes. In diesem Fall ist es zweckmäßig, die Sollbruchstellen sowohl in Längsrichtung der Streckung der Gefäßstütze als auch quer dazu anzuordnen, um einen Bereich öffnen zu können.

Soweit die erfindungsgemäßen Gefäßstützen mit einer Membran versehen sind, die mit der Gefäßstütze vernäht ist, können Fäden aus resorbierbarem Material oder Reineisen- oder Wolframdraht verwandt werden. Die Membran selbst kann aus einem biologischen oder synthetischen Material bestehen. Letzteres kann durch Elektrospinnen appliziert worden sein.

Um bei den erfindungsgemäßen Gefäßstützen die Längenreduktion bei der Aufweitung in Grenzen zu halten, können eine Mehrzahl von Ringsegmenten über spiralförmige, s-förmige oder bogenförmige Verbindungselemente mit benachbarten Ringelementen verbunden sein, die sich bei der Dilatation strecken. Bevorzugt in diesem Zusammenhang sind ferner paarweise in gegensätzlicher Richtung angeordnete bogenförmige Verbindungselemente, die zusammen mit den angrenzenden Streben der Ringelemente ein sternförmiges Element ergeben. Dadurch ergibt sich trotz hoher Flexibilität durch die multizelluläre Struktur eine hohe Radialfestigkeit, die durch spiralige oder abwechselnde Anordnung von einem oder mehreren sternförmigen Segmenten noch erhöht wird. Auch durch derartige Ausbildungen wird das Biegeverhalten der Gefäßstütze insgesamt günstig beeinflusst.

Die Verbindungselemente sind dergestalt ausgeführt, dass der Bruch der Sollbruchstellen über einen Hebel im Verbindungselement herbeigeführt wird. Der Steg ist am Verbinder dann beispielsweise in Form einer zweidimensionalen Gelenkkugel und Gelenkpfanne angebracht, wobei beide Elemente durch einen Verbindungssteg - der Sollbruchstelle - zwischen zweidimensionaler Gelenkkugel und -pfanne verbunden sind. Dies hat den Vorteil, dass die Ausführung der Sollbruchstelle stabiler sein kann, da die auf diese Stelle bei Überdehnung eine Hebelkraft wirkt. Dieser Bereich weist entsprechend beim nicht überdehnten Stent eine höhere Festigkeit auf. Eine Überdehnung führt dabei ebenfalls und gegebenenfalls sogar leichter und in jedem Fall aber definiert zu einem Bruch der Sollbruchstelle. Tatsächlich ist es möglich, die zum Bruch der Sollbruchstelle notwendige Kraft in einem gegebenen Rahmen durch Wahl des Hebels vorzugeben. Vorteilhaft ist auch, dass eine solche Ausführung der Sollbruchstelle über einen Hebel eine präzise Winkelbegrenzung der maximal möglichen Aufdehnung des Stents ohne Bruch in den entsprechenden Verbindern erlaubt, was durch vorgegebene Einrastpunkte zusätzlich definiert werden kann. Die Stege der Verbindungselemente zwischen den einzelnen Ringelementen weisen vorzugsweise einen etwas geringeren Querschnitt auf als die Stege der mäandrierenden oder zickzackförmigen Ringelemente. Der Stegquerschnitt der Verbindungstege beträgt dann etwa 60 bis 80% des Querschnitts der Stege der Ringelemente. Es sind beliebige Kombinationsmöglichkeiten mit den verschiedenen Formen von Stegen der Verbindungselemente denkbar.

Soweit die Sollbruchstellen aus Einkerbungen bestehen, haben diese in der Regel eine Tiefe von 30 bis 50% der Stegdicke der Verbindungselemente, auf denen sie angeordnet sind. Perforationen, die in der Regel linear angeordnet sind, haben einen Durchmesser etwa von 20 bis 60% der Stegbreite der entsprechenden Verbindungselemente. Die Perforationen können rund, dreieckig, rechteckig oder sternförmig ausgebildet sein.

Als Material für die erfindungsgemäße Gefäßstütze können übliche Materialien verwandt werden, insbesondere biokompatible Metalle wie Eisen, Stahl, Wolfram, Niob, Platin, Titan, Legierungen von Nickel und Titan sowie Legierungen mit mindestens einem dieser Metalle, wie Platin-Iridium. Soll die Gefäßstütze selbst expandierbar sein, wird vorzugsweise eine durch Wärmebehandlung temperaturoptimierte Nickel-Titan-Legierung verwandt.

Die Gefäßstütze kann zur Verbesserung des Einwachsens in die Gefäßwand mit einem biokompatiblen Material oder mit geeigneten Medikamenten zur Vermeidung von Hyperproliferation der Gefäßwand beschichtet sein oder durch Bestrahlung oder radioaktiven Zerfall eine Strahlung freisetzen.

Ferner kann die Gefäßstütze oder die Bespannung auch aus resorbierbaren Kunststoffen, z. B. aliphatischen Polyestern wie Polydioxanon, bestehen.

Die erfindungsgemäßen Gefäßstützen werden vorzugsweise aus nahtlos gezogenen Rohren gebildet, um Verspannungen und Risse zu vermeiden, wie das im Bereich von Schweißnähten häufig der Fall ist. Die Strukturen werden vorzugsweise durch Laser- oder Wasserstrahlschneiden, Elektroerosion und Elektropolitur hergestellt. Die Gefäßstützen haben im Erstehungszustand eine gitterförmige Struktur, die durch Crimpen auf einen Dilatationsballon komprimiert werden kann und nach der Implantation durch Dilatation über den ursprünglichen Zustand nach der Herstellung hinaus aufgeweitet werden kann. Die Sollbruchstellen werden in der Regel erst mit einer Nach- oder Redilatation aktiviert und zum Aufreißen gebracht. Je nach Verwendung der erfindungsgemäßen Gefäßstütze mit ihren verschiedenen Ausführungsformen kann in speziellen Fällen auch auf Sollbruchstellen verzichtet werden.

Sofern die Verbindungselemente als reine Klemmvorrichtungen für Membranen angelegt sind, kann in diesen speziellen Fällen auf Sollbruchstellen innerhalb der Klemmen verzichtet werden.

Weitere Merkmale und Vorteile der Erfindung ergeben sich aus der nachfolgenden Beschreibung von Ausführungsbeispielen anhand der Abbildungen, die erfindungswesentliche Einzelheiten zeigen sowie aus den Ansprüchen. Die einzelnen Merkmale können hier einzeln für sich oder zu mehreren beliebigen Kombinationen bei Varianten der Erfindung verwirklicht sein. Es versteht sich, dass Abbildungen nur Beispiele sind und dass jedes in einer Abbildung gezeigte besondere Merkmal in beliebiger Kombination mit Merkmalen aus anderen Abbildungen zur Erfindung gehört.

Von den Abbildungen zeigen:
- Fig.1:: Eine Seitenansicht einer Ausführungsform der Erfindung, die auf einen Dilatationsballon gecrimpt wurde;
- Fig. 2:: eine Seitenansicht der gleichen Ausführungsform, wie Fig. 1 in einem ersten aufgedehnten Zustand, nachdem der Dilatationsballon entfernt wurde;
- Fig. 3:: in einer weiteren Seitenansicht die Ausführungsform von Fig. 1 und 2 bei der maximalen Aufdehnung mit einem druckfesten Dilatationsballon mit einem größeren Durchmesser und aufgebrochenen Sollbruchstellen;
- Fig. 4:: in einer Seitenansicht die Ausführungsform der Erfindung gemäß Fig. 2 bei Durchführung einer Fenestrierung;
- Fig. 5:: die Ausführungsform gemäß Fig. 2, gecovert mit einer membranartigen Folie;
- Fig. 6:: Verbindungselemente, wie sie erfindungsgemäß zum Einsatz kommen;
- Fig. 7:: eine Gefäßstütze in ausgebreitetem Zustand mit einem durch Verbindungstege mit Sollbruchstellen definierten Bereich für Fenestrierungen;
- Fig. 8:: eine Variante einer erfindungsgemäßen Gefäßstütze mit verlängerten Stegen im zentralen Bereich;
- Fig. 9:: eine erfindungsgemäße Gefäßstütze mit über die Endsegmente fixierbarer Membran;
- Fig. 10:: eine weitere Variante der Ausführungsform von Fig. 14;
- Fig. 11:: Varianten von Klemmelementen, mit denen eine Membran auf der Gefäßstütze fixiert werden kann; und
- Fig. 12:: eine erfindungsgemäße Gefäßstütze, die beispielsweise für Herzklappenimplantate eingesetzt werden kann.
- Fig. 13:: eine weitere erfindungsgemäße Gefäßstütze, die beispielsweise für Herzklappenimplantate eingesetzt werden kann.
- Fig. 14:: die erfindungsgemäße Gefäßstütze gemäß Figur 14 mit einer eingespannten Membran mit Herzklappe.
- Fig. 15:: eine weitere Variante einer erfindungsgemäßen Gefäßstütze, die beispielsweise für Herzklappenimplantate eingesetzt werden kann und zusätzliche Klemmelemente für eine weitere Membran aufweist.

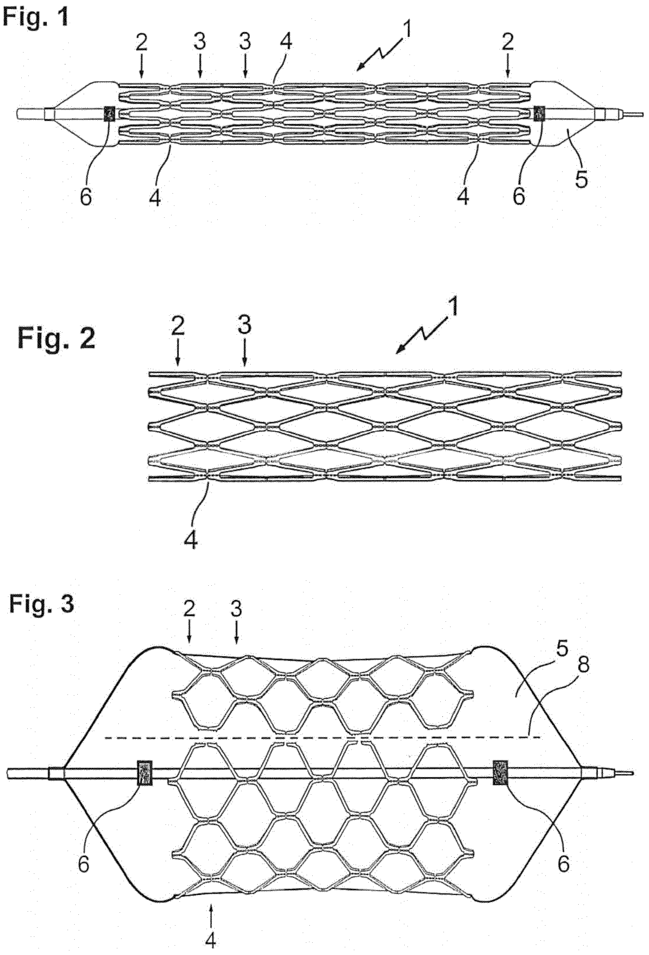

Figur 1 zeigt eine Seitenansicht einer Ausführungsform der Erfindung, die auf einen Dilatationsballon gecrimpt wurde. Die Gefäßstütze 1 besteht aus einer Vielzahl von Ringelementen 2 und 3, die durch Verbindungselemente 4 miteinander verbunden sind. Ringelemente 2 sind randständig und haben jeweils nur einen Nachbarn, während Ringelemente 3 entweder ein randständiges oder ein innenständiges Ringelement zum Nachbarn haben. Die Verbindungselemente 4 sind Verbindungsstege, die von den äußeren Wendepunkten der Ringelemente ausgehen und im dargestellten Fall zu den äußeren Wendepunkten des benachbarten Ringelements 3 verlaufen.

Die Stege der Verbindungselemente 4 weisen in Richtung der Längsachse der Gefäßstütze 1 verlaufende Perforationen auf, die eine Schwächungszone ausbilden, die zur einer Sollbruchstelle führt. Die Perforationen setzen sich durch die angrenzenden Teile der Stege der Ringsegmente 2, 3 hindurch fort.

Die Gefäßstütze 1 ist auf einen handelsüblichen Ballon 5 aufgebracht, der mittels Katheter und Führungsdraht an den Einsatzort transportiert werden kann.

Figur 2 zeigt die Gefäßstütze von Figur 1 in einem ersten implantierten Zustand mit leicht aufgeweiteten Ringsegmenten 2, 3 und intakten Verbindungselementen 4. Der Dilatationsballon ist aus der Gefäßstütze 1 entfernt.

Figur 3 schließlich zeigt die Gefäßstütze von Figur 1 und 2 auf einem Dilatationsballon 5. In diesem hat der Dilatationsballon einen größeren Radius und kann damit die Gefäßstütze 1 weiter aufspreizen, bis zum Aufreißen bzw. Aufbrechen der Perforationslinie 8, die parallel zur Längsachse der Gefäßstütze 1 verläuft.

Zur exakten Positionierung des Implantats bzw. des Dilatationsballons ist dieser mit Markern 6 ausgestattet, die proximal und distal zur Gefäßstütze 1 angeordnet sind.

Die in Figur 3 dargestellte Seitenansicht der Gefäßstütze 1 mit Aufweitung auf einen maximalen Durchmesser und aufgebrochenen Verbindungselementen 4 entlang der Perforationslinie 8, die parallel zur Längsachse der Gefäßstütze 1 verläuft, führt zwar zu einer Gefäßstütze 1 mit reduzierter Radialkraft, erlaubt aber danach ein weiteres Wachstum des Durchmessers des entsprechenden Gefäßes, etwa der Aorta oder der Pulmonalarterie eines im Wachstum befindlichen Kindes. Gegebenenfalls kann in die aufgebrochene Gefäßstütze 1 eine weitere Gefäßstütze mit einem größeren maximalen Enddurchmesser implantiert werden.

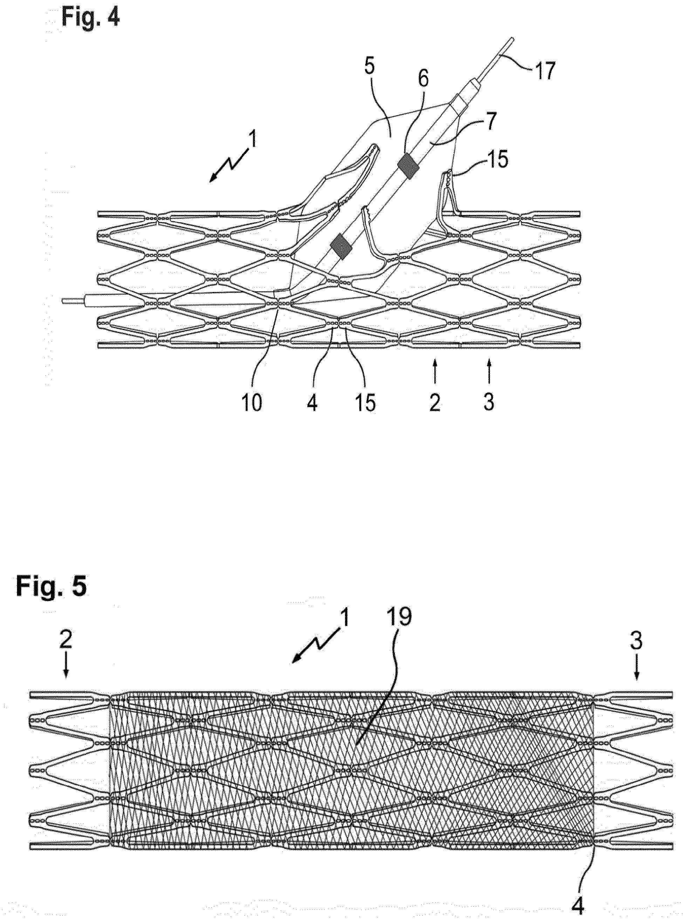

Figur 4 zeigt die Gefäßstütze 1 von Figur 2 und einen darin eingeführten Dilatationsballon 5, der seitlich durch die Wand der Gefäßstütze 1 hindurchgeführt und dilatiert worden ist. Entsprechend sind die Sollbruchstellen der Verbindungsstege der betroffenen Ringelemente 2 und 3 aufgebrochen, so dass ein Fenster entsteht, durch das auch ein abzweigendes Gefäß mit Blut versorgt werden kann.

Deutlich dargestellt sind die in Längsrichtung der Gefäßstütze 1 verlaufenden Perforationslinien 15 sowie Einkerbungen 10, die quer dazu durch die Verbindungsstege 4 verlaufen und eine Auftrennung der Gefäßstütze quer zur Längsrichtung erlauben.

Der Dilatationsballon 5 an der Spitze des Katheters 7 wird über einen Führungsdraht 17 vorgeschoben und gezielt in den bereits in den Bereich einer Gefäßabzweigung implantierten Stent 1 positioniert, so dass dieser im Bereich der Abzweigung aufgebrochen wird.

Figur 5 zeigt den Stent von Figur 2 mit einer darüber gespannten schlauchförmigen Membran 19, die beispielsweise dazu dient, eine Gefäßanomalie, etwa ein Aneurysma oder eine Perforation, abzuschirmen. Die Membran 19 kann beispielsweise aus Teflon oder einem anderen körperverträglichen Material bestehen und gegebenenfalls mit einem Medikament ausgestattet sein.

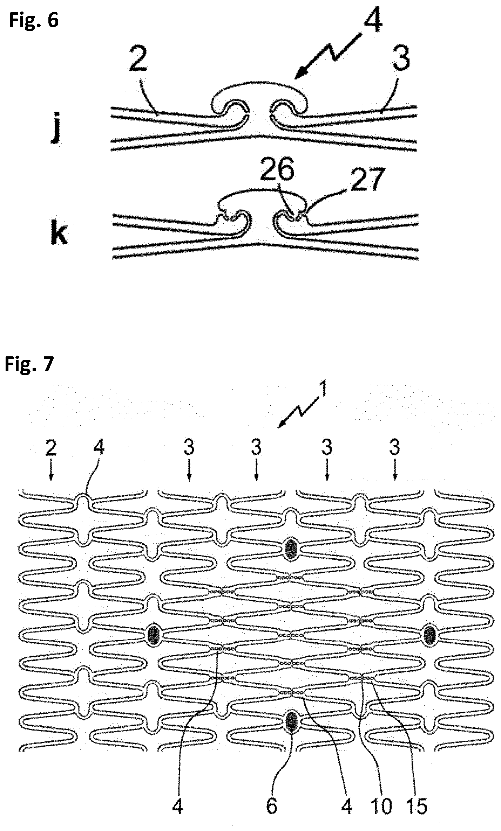

Figur 6 zeigt erfindungsgemäße Verbindungselemente 4 zwischen Ringelementen 2, 3. Die Darstellungen j und k zeigen erfindungsgemäße Ausführungsformen, bei denen die Stentstruts im Verbindungselement ähnlich einer Gelenkverbindung angeordnet und über einen Verbindungssteg verbunden sind. Diese Anordnung ermöglicht die Einstellung von definierten Hebelwirkungen im Verbindungselement bei Aufdehnung des Stents. Darstellung j zeigt eine einfache Ausführungsform, in Darstellung k wird durch die spezielle Anordnung ein Knickpunkt 27 an der zweidimensionalen Gelenkkugel 26 vorgegeben.

Figur 7 zeigt eine Ausführungsform einer erfindungsgemäßen Gefäßstütze 1 in ausgebreitetem Zustand mit einer Teilfläche, innerhalb derer die Verbindungselemente 4 zwischen Ringelementen 3 mit Sollbruchstellen versehen sind.

Die Zone, die für die Fenestrierung vorgesehen ist, ist durch vier Marker 6 eingegrenzt. Zwischen diesen Markern 6 sind alle Verbindungselemente 4 mit einer Sollbruchstelle durch aneinander gereihte Perforationslöcher 15 in Längsrichtung und mit Kerben zur Generierung einer Sollbruchstelle in Umfangsrichtung ausgestattet. Bei Expansion eines Katheterballons im Bereich dieser Fenestrierungszone reißen die Verbindungselemente 4 entlang der Sollbruchstellen auf und öffnen das Fenster, beispielsweise in ein Seitengefäß hinein.

Die Gefäßstütze gemäß Figur 7 weist in den Bereichen, in denen keine Sollbruchstellen in den Verbindungselementen 4 vorgesehen sind, die gekrümmten Verbindungselemente 4, auf die paarweise mit ihren offenen Seiten einander zugewandt sind und mit den angrenzenden Schlingen der Ringelemente 2, 3 ein sternförmiges Design ergeben, das für eine gute Radialkraft steht.

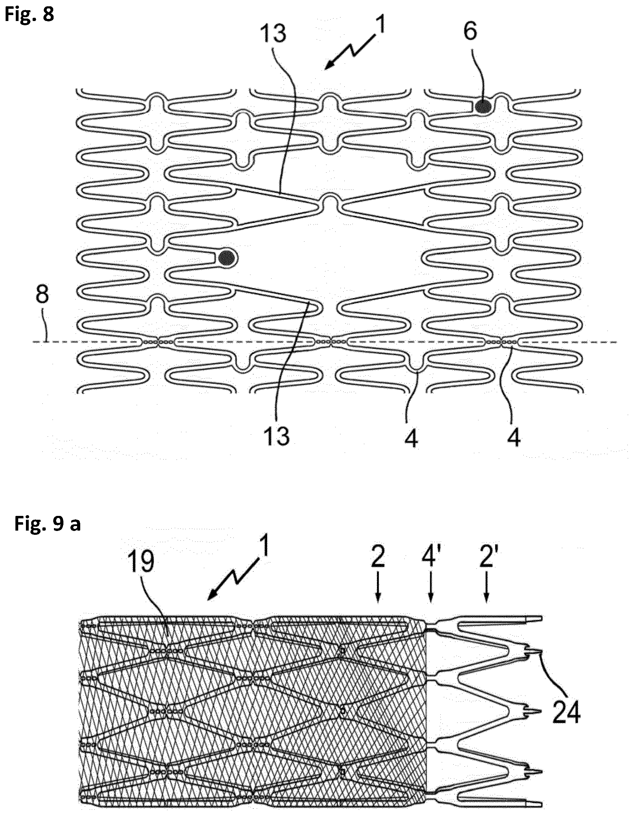

Figur 8 zeigt eine erfindungsgemäße Gefäßstütze 1 mit im zentralen Bereich stark verlängerten Stegen 13, um so im dilatierten Zustand einen verbesserten Seitenastzugang zu ermöglichen. Der Stent 1 ist entlang der Trennlinie 8 längs auftrennbar, wobei entlang dieser Trennlinie die Verbindungselemente 4 zwei Durchbrechungen aufweisen, die die für die Auftrennung nötige Schwächung herbeiführen.

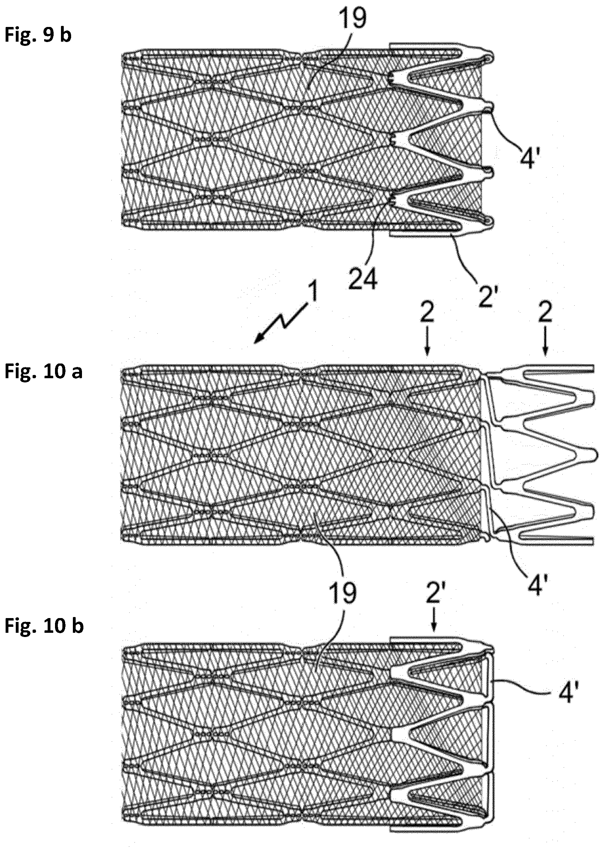

Figur 9a/b zeigt eine erfindungsgemäße Gefäßstütze mit einer Bespannung 19 und Sollbruchstellen in den Verbindungselementen 4, die sowohl in Längsrichtung als auch quer dazu verlaufen. Zur Fixierung der Membran 19 weist der Stent 1 ein Ringelement 2' auf, das über Verbindungsstege 4' an das randständige Ringelement 2 des Stents 1 angebunden ist und auf die Membran 19 zurückgeklappt werden kann. Befestigungsdorne 24 dienen der Fixierung der Membran 19.

Figur 10a/b zeigt eine Variante der Ausführungsform von Figur 9a/b, bei der das endständige Ringsegment 2' über seitlich versetzte Verbindungselemente 4' mit den randständigen Ringsegmenten 2 verbunden ist. Die schräg seitlich verlaufenden Verbindungselemente 4' erleichtern das Umklappen des Ringelements 2' auf die Membran 19.

Die Ausführungsformen gemäß Figur 9 wie 10 weisen in dem unter der Membran 19 liegenden Teil des Stents 1 die erfindungsgemäßen Sollbruchstellen an den Verbindungselementen 4 auf.

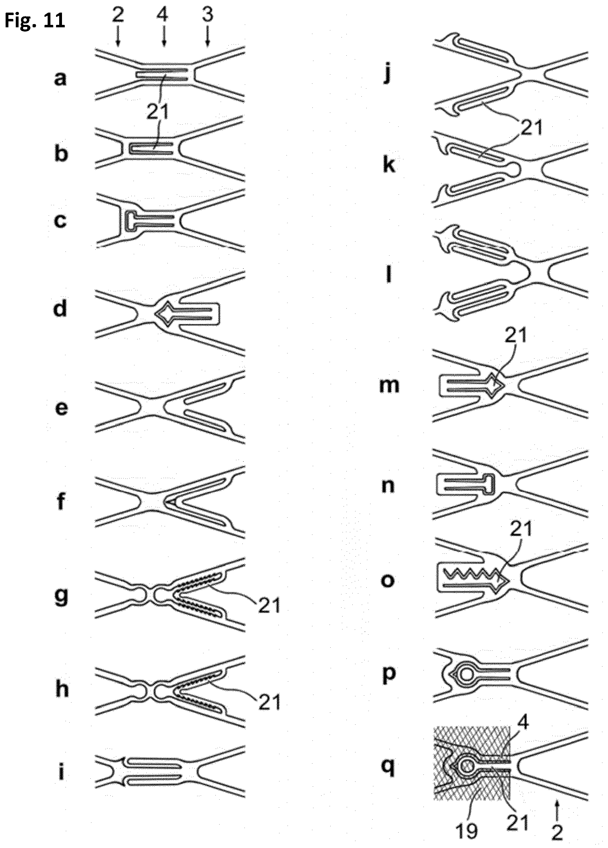

Figur 11 zeigt in den Varianten a bis p eine Vielzahl von Klemmelementen 21, die der Fixierung einer Membran dienen. Die Klemmelemente 21 sind aus einem Verbindungselement 4 oder einem Ringelement 2, 3 ausgeklinkte Stege, die gegen das Verbindungselement oder Ringelement federnd beweglich sind. Die Membran kann damit unter dieses federnde Klemmelement 21 geschoben und dadurch fixiert werden.

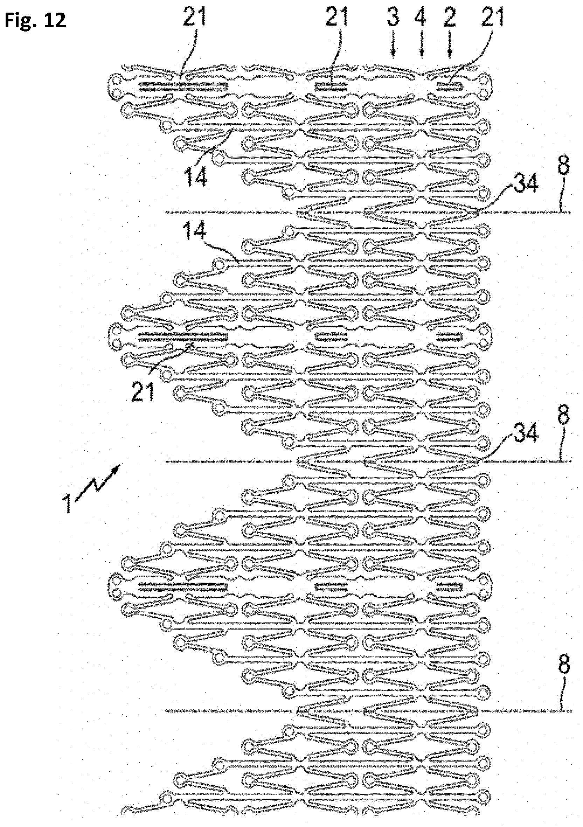

Die Klemmelemente 21 können vorteilhaft sowohl aus dem Verbindungselement 4 wie auch aus einem Ringelement 2 oder 3 ausgeklinkt werden. Es handelt sich dabei um freibewegliche Stege mit nahezu beliebiger Form. Ein verbreitertes Ende in Form eines Sterns, Hammerkopfes oder einer Scheibe, einer Zähnung oder Dornen dienen der besseren Befestigung einer Membran. Die Klemmelemente 21 können paarweise auftreten und verlaufen im Wesentlichen in Längsrichtung der Gefäßstütze. In jedem Fall wird eine Membran durch Einschieben zwischen die Gitterstruktur des Stents und den freibeweglichen Stegen der Klemmelemente 21 fixiert, siehe Figur 12q.

Figur 12 zeigt eine Gefäßstütze 1 mit einer für Herzklappen typischen Stentstruktur mit randständigen Ringsegmenten 2, daran angrenzenden Ringsegmenten 3 sowie Verbindungsstegen 4. Verbindungsstege sind hier vielfach als gerade Stege ausgebildet, die über die gesamte Länge des Stents 1 verlaufen und damit dessen Länge definieren.

Im dargestellten Fall sind Verbindungselemente 34 im Bereich der Bruchlinien 8 zwischen Stegen der Ringelemente 2 und 3 angeordnet. Die Sollbruchstellen werden durch Perforationslinien gebildet, die bei Nachdilatation aufreißen und damit eine Anpassung der Klappe an die wachsenden Gefäße erlauben.

Der in Figur 12 dargestellte Stent 1 weist federnde Stege 21 auf, die sich zur Fixierung eines Herzklappen tragenden Stents in der umgebenden Gefäßwand eignen und so ein Verrutschen des Stents verhindern. Ferner kann dieser Stent auch weitere Klemmelemente wie sie in Figur 11 a bisq dargestellt sind enthalten, damit das Klappenmaterial eingeklemmt werden kann. Auf diese Weise entfällt das sonst übliche Verkleben, Verdrahten oder einnähen der festen oder beweglichen Klappenteile.

Zur Verringerung von paravalvulären Leckagen kann so vorteilhaft einfach auch noch eine zweite polsterartige Membran nur zur Abdichtung am Unterrand des Stents 1 mit geeigneten Klemmelementen 21 innen und außen eingeklemmt werden.

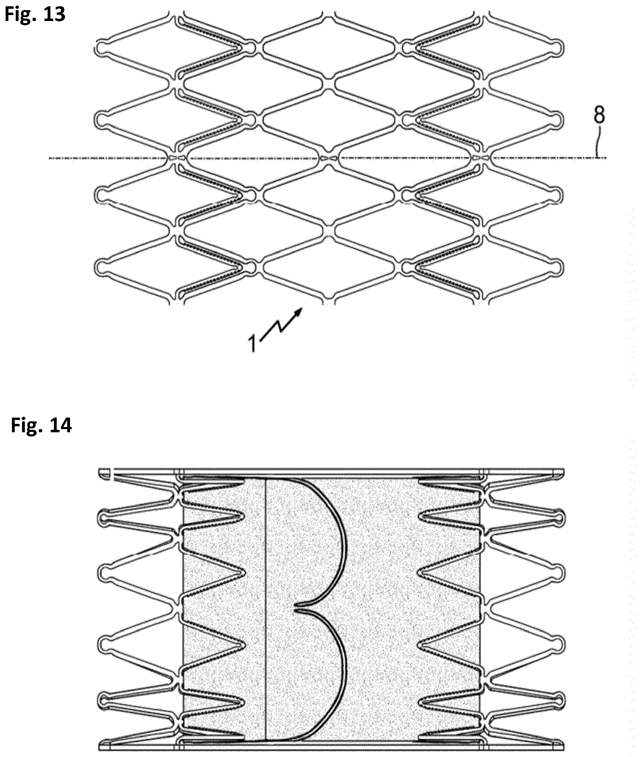

Figur 13 zeigt eine weitere Ausführungsform einer Gefäßstütze, die Klemmvorrichtungen zur Fixierung einer Herzklappen-tragenden Membran aufweist. Die Klemmvorrichtungen können einer der in Figur 12 dargestellten Variante oder auch einer Kombination dieser oder sonstiger erfindungsgemäßer Klemmvorrichtungen entsprechen.

Figur 14 zeigt beispielhaft, wie eine Herzklappen-tragende Membran in eine Gefäßstütze, wie in Figur 14 dargestellt, eingeklemmt wird.

Figur 15 zeigt eine weitere Ausführungsform einer Gefäßstütze, die neben Klemmvorrichtungen zur Fixierung einer Herzklappen-tragenden Membran weitere Klemmvorrichtungen zur Aufnahme einer weiteren, der Außenseite der Gefäßstütze aufliegenden Membran aufweist. Zusätzlich befinden sich an den nach außen weisenden Bögen der randständigen Ringelemente Ösen, durch die beispielsweise ein oder mehrere Fäden gezogen werden können, um eine bereits expandierte Gefäßstütze beispielsweise im Rahmen einer Explantation oder Repositionierung wieder in einen Katheter zurückzuziehen zu können.

## Patentansprüche

1. Radial aufweitbare Gefäßstütze (1), welche eine Mehrzahl von miteinander flexibel verbundenen mäandrierenden und aus Stegen aufgebauten Ringelementen (2, 3) aufweist, welche eine Gefäßstütze (1) mit einem proximalen und einem distalen Ende und einer Längsachse definieren, wobei die Ringelemente (2, 3) entlang der Längsachse der Gefäßstütze nebeneinander angeordnet sind und aneinander grenzende Ringelemente (2, 3) durch Verbindungselemente (4) miteinander verbunden sind, wobei zwischen wenigstens zwei aneinander grenzenden Ringelementen (2, 3) Verbindungselemente (4) angeordnet sind, die jeweils mindestens ein aufdehnbares Erweiterungselement mit Sollbruchstelle (10) aufweisen, wobei die Erweiterung in Längs- und/oder in Querrichtung möglich ist und die Sollbruchstellen (10) erst nach einer Überdehnung des Erweiterungselements über dessen maximalen Erweiterungspunkt hinaus brechen, **dadurch gekennzeichnet, dass** die Stege zumindest jeweils eines Ringelements (2, 3) im Verbindungselement (4) ähnlich einer zweidimensionalen Gelenkverbindung mit einer Gelenkkugel und einer Gelenkpfanne angeordnet und über eine Sollbruchstelle (10) mit dem Verbindungselement (4) verbunden sind und der Bruch der Sollbruchstelle (10) über einen Hebel im Verbindungselement (4) herbeigeführt wird.

2. Gefäßstütze nach Anspruch 1, **gekennzeichnet durch** Verbindungsstege innerhalb der Verbindungselemente (4), die so angeordnet sind, dass sich die Erweiterungselemente stufenweise erweitern lassen.

3. Gefäßstütze nach Anspruch 1 oder 2, **dadurch gekennzeichnet, dass** die Sollbruchstellen (10) an den Verbindungselementen (4) in Richtung der Längs- oder Querachse der Gefäßstütze oder helixförmig verlaufen.

4. Gefäßstütze nach einem der vorstehenden Ansprüche, **dadurch gekennzeichnet, dass** die Sollbruchstellen (10) Einkerbungen sind, die 20 bis 70% der Stegdicke des Verbindungselements (4) ausmachen.

5. Gefäßstütze nach einem der Ansprüche 1 bis 3, **dadurch gekennzeichnet, dass** die Sollbruchstellen (10) entlang einer Linie verlaufende Perforationslöcher sind, deren Durchmesser 20 bis 70% der Stegbreite der Verbindungselemente (4) ausmacht.

6. Gefäßstütze nach einem der vorstehenden Ansprüche, **dadurch gekennzeichnet, dass** die Verbindungselemente (11) als Dehnungselemente (11) bogenförmig ausgebildet sind, wobei zwei aneinander grenzende gegenläufige Dehnungselemente (11) mit den angrenzenden Ringelementen (2, 3) ein sternförmiges Segment bilden.

7. Gefäßstütze nach einem der vorstehenden Ansprüche, **dadurch gekennzeichnet, dass** die quer zur Längsrichtung verlaufenden Sollbruchstellen (10) um die Zirkumferenz des Stents herum ausgerichtet sind, um den Stent ganz oder teilweise zu teilen.

8. Gefäßstütze nach einem oder mehreren der vorstehenden Ansprüche, **dadurch gekennzeichnet, dass** die Sollbruchstellen (10) dergestalt angeordnet sind, dass sie das Aufbrechen eines Fensters in der Gefäßstütze ermöglichen.

9. Gefäßstütze nach Anspruch 8, **gekennzeichnet durch** gegenüberliegende Marker im Bereich der möglichen Fenistrierungen.

10. Gefäßstütze nach einem der vorstehenden Ansprüche mit einer darauf aufgebrachten Membran, die vorzugsweise das äußere Ringelement (2) freilässt.

11. Gefäßstütze nach Anspruch 10, **dadurch gekennzeichnet, dass** die Membran zwischen dem äußeren Ringelement (2) und dem anliegenden Ringelement (3) durch Umklappen des Ringelements (2) fixiert werden kann.

12. Gefäßstütze nach Anspruch 11, **dadurch gekennzeichnet, dass** die Membran durch Elektrospinnen erzeugt ist.

13. Gefäßstütze nach Anspruch 10, **dadurch gekennzeichnet, dass** die Membran schlauchförmig ausgebildet und durch Elektrospinnen erzeugt ist und durch Klemmelemente insbesondere an den Ringelementen (2, 3) fixiert ist.

14. Gefäßstütze nach Anspruch 13, **dadurch gekennzeichnet, dass** die Ringelemente (2, 3) eine zweite Reihe Klemmelemente aufweisen, wobei an den beiden inneren Reihen von Klemmelementen eine erste Membran im Inneren der Gefäßstütze (1) befestigt werden kann und an den beiden äußeren Reihen von Klemmelementen ein zweite Membran an der Außenseite der Gefäßstütze (1) befestigt werden kann.

15. Gefäßstütze nach einem der Ansprüche 10 bis 14, **dadurch gekennzeichnet, dass** mindestens eine Membran 2 oder 3 klappenartige Strukturen aufweist und durch Nähte, verkleben und/oder mit Klemmelementen an der Gefäßstütze (1) befestigt ist, wobei die Gefäßstütze im unteren Bereich eine zweite polsterförmige Membran aufweisen kann.

16. Gefäßstütze nach Anspruch 15, **dadurch gekennzeichnet, dass** sich an den nach außen weisenden Bögen der randständigen Ringelemente Ösen beispielweise zur Aufnahme eines oder mehrerer Fäden befinden, um eine bereits expandierte Gefäßstütze wieder in einen Katheter zurückziehen zu können.

17. Gefäßstütze nach einem der vorstehenden Ansprüche, **dadurch gekennzeichnet, dass** sie zumindest in Teilbereichen mit einer antiproliferativen oder gerinnungshemmenden Beschichtung versehen ist.

## Claims

1. Radially expandable vascular support (1), which has a plurality of meandering ring elements (2, 3) which are flexibly connected to one another and are composed of webs and which define a vascular support (1) with a proximal and a distal end and a longitudinal axis, the ring elements (2, 3) being arranged next to one another along the longitudinal axis of the vascular support and adjoining ring elements (2, 3) being connected to one another by connecting elements (4), at least two adjoining ring elements (2, 3) being connected to one another by connecting elements (4), 3) are arranged next to one another along the longitudinal axis of the vascular support and adjoining ring elements (2, 3) are connected to one another by connecting elements (4), connecting elements (4) being arranged between at least two adjoining ring elements (2, 3), each of which has at least one expandable widening element with a predetermined breaking point (10), wherein the expansion is possible in the longitudinal and/or transverse direction and the predetermined breaking points (10) only break after overstretching of the expansion element beyond its maximum expansion point, **characterised in that** the webs of at least one ring element (2, 3) in each case are arranged in the connecting element (4) similarly to a two-dimensional joint connection with a joint ball and a joint socket and are connected to the connecting element (4) via a predetermined breaking point (10) and the breaking of the predetermined breaking point (10) is brought about via a lever in the connecting element (4).

2. Vascular support according to claim 1, **characterised by** connecting webs within the connecting elements (4), which are arranged in such a way that the extension elements can be extended in stages.

3. Vascular support according to claim 1 or 2, **characterised in that** the predetermined breaking points (10) on the connecting elements (4) extend in the direction of the longitudinal or transverse axis of the vascular support or helically.

4. Vascular support according to one of the preceding claims, **characterised in that** the predetermined breaking points (10) are notches which make up 20 to 70% of the web thickness of the connecting element (4).

5. Vascular support according to one of claims 1 to 3, **characterised in that** the predetermined breaking points (10) are perforation holes running along a line, the diameter of which accounts for 20 to 70% of the web width of the connecting elements (4).

6. Vascular support according to one of the preceding claims, **characterised in that** the connecting elements (11) are designed as arcuate expansion elements (11), wherein two adjoining expansion elements (11) running in opposite directions form a star-shaped segment with the adjoining ring elements (2, 3).

7. Vascular support according to one of the preceding claims, **characterised in that** the predetermined breaking points (10) extending transversely to the longitudinal direction are aligned around the circumference of the stent in order to divide the stent completely or partially.

8. Vascular support according to one or more of the preceding claims, **characterised in that** the predetermined breaking points (10) are arranged in such a way that they enable a window in the vascular support to be broken open.

9. Vascular support according to claim 8, **characterised by** opposing markers in the area of the possible fenestrations.

10. Vascular support according to one of the preceding claims with a membrane applied thereto, which preferably leaves the outer ring element (2) free.

11. Vascular support according to claim 10, **characterised in that** the membrane can be fixed between the outer ring element (2) and the adjacent ring element (3) by folding back the ring element (2).

12. Vascular support according to claim 11, **characterised in that** the membrane is produced by electrospinning.

13. Vascular support according to claim 10, **characterised in that** the membrane is tubular and is produced by electrospinning and is fixed in particular to the ring elements (2, 3) by clamping elements.

14. Vascular support according to claim 13, **characterised in that** the ring elements (2, 3) have a second row of clamping elements, wherein a first membrane can be fastened to the inside of the vascular support (1) at the two inner rows of clamping elements and a second membrane can be fastened to the outside of the vascular support (1) at the two outer rows of clamping elements.

15. Vascular support according to one of claims 10 to 14, **characterised in that** at least one membrane has 2 or 3 flap-like structures and is attached to the vascular support (1) by sutures, adhesive bonding and/or with clamping elements, wherein the vascular support can have a second cushion-shaped membrane in the lower region.

16. Vascular support according to claim 15, **characterised in that** eyelets, for example for receiving one or more threads, are located on the outwardly pointing arcs of the edge ring elements in order to be able to pull an already expanded vascular support back into a catheter.

17. Vascular support according to one of the preceding claims, **characterised in that** it is provided at least in partial areas with an antiproliferative or anticoagulant coating.

## Revendications

1. Support vasculaire (1) pouvant être dilaté radialement, qui présente une pluralité d'éléments annulaires (2, 3) à méandres et constitués d'entretoises, reliés entre eux de manière flexible, qui définissent un support vasculaire (1) avec une extrémité proximale et une extrémité distale et un axe longitudinal, les éléments annulaires (2, 3) sont disposés les uns à côté des autres le long de l'axe longitudinal du support vasculaire et des éléments annulaires (2, 3) adjacents sont reliés les uns aux autres par des éléments de liaison (4), des éléments de liaison (4) étant disposés entre au moins deux éléments annulaires (2, 3) adjacents, lesquels présentent chacun au moins un élément d'extension extensible avec un point destiné à la rupture (10), l'extension étant possible dans le sens longitudinal et/ou dans le sens transversal et les points destinés à la rupture (10) ne se rompant qu'après une sur-extension de l'élément d'extension au-delà de son point d'extension maximal, **caractérisé en ce que** les entretoises d'au moins respectivement un élément annulaire (2, 3) sont disposées dans l'élément de liaison (4) de manière similaire à une liaison articulée bidimensionnelle avec une rotule d'articulation et une cavité articulaire et sont reliées à l'élément de liaison (4) par un point destiné à la rupture (10) et la rupture du point destiné à la rupture (10) est provoquée par un levier dans l'élément de liaison (4).

2. Support vasculaire selon la revendication 1, **caractérisé par** des entretoises de liaison à l'intérieur des éléments de liaison (4), qui sont disposées de telle sorte que les éléments d'extension peuvent être élargis par étapes.

3. Support vasculaire selon la revendication 1 ou 2, **caractérisé en ce que** les points destinés à la rupture (10) sur les éléments de liaison (4) s'étendent dans la direction de l'axe longitudinal ou transversal du support vasculaire ou en forme d'hélice.

4. Support vasculaire selon l'une des revendications précédentes, **caractérisé en ce que** les points destinés à la rupture (10) sont des encoches qui représentent 20 à 70% de l'épaisseur de l'âme de l'élément de liaison (4).

5. Support vasculaire selon l'une des revendications 1 à 3, **caractérisé en ce que** les points de rupture (10) sont des trous de perforation s'étendant le long d'une ligne, dont le diamètre représente 20 à 70% de la largeur de l'âme des éléments de liaison (4).

6. Support vasculaire selon l'une des revendications précédentes, **caractérisé en ce que** les éléments de liaison (11) sont réalisés sous forme d'éléments de dilatation (11) en forme d'arc, deux éléments de dilatation (11) adjacents et tournant en sens inverse formant un segment en forme d'étoile avec les éléments annulaires (2, 3) adjacents.

7. Support vasculaire selon l'une des revendications précédentes, **caractérisé en ce que** les points de rupture (10) s'étendant transversalement à la direction longitudinale sont orientés autour de la circonférence du stent afin de diviser le stent en tout ou partie.

8. Support vasculaire selon une ou plusieurs des revendications précédentes, **caractérisé en ce que** les points de rupture (10) sont disposés de manière à permettre la rupture d'une fenêtre dans le support vasculaire.

9. Support vasculaire selon la revendication 8, **caractérisé par** des marqueurs opposés dans la zone des fenestrations possibles.

10. Support vasculaire selon l'une des revendications précédentes avec une membrane appliquée sur celui-ci, qui laisse de préférence l'élément annulaire extérieur (2) libre.

11. Support vasculaire selon la revendication 10, **caractérisé en ce que** la membrane peut être fixée entre l'élément annulaire extérieur (2) et l'élément annulaire adjacent (3) en rabattant l'élément annulaire (2).

12. Support vasculaire selon la revendication 11, **caractérisé en ce que** la membrane est produite par électrofilage.

13. Support vasculaire selon la revendication 10, **caractérisé en ce que** la membrane est de forme tubulaire et est produite par électrofilage et est fixée par des éléments de serrage, en particulier sur les éléments annulaires (2, 3).

14. Support vasculaire selon la revendication 13, **caractérisé en ce que** les éléments annulaires (2, 3) comprennent une deuxième rangée d'éléments de serrage, une première membrane pouvant être fixée à l'intérieur du support vasculaire (1) sur les deux rangées intérieures d'éléments de serrage et une deuxième membrane pouvant être fixée à l'extérieur du support vasculaire (1) sur les deux rangées extérieures d'éléments de serrage.

15. Support vasculaire selon l'une des revendications 10 à 14, **caractérisé en ce qu'**au moins une membrane présente 2 ou 3 structures en forme de rabat et est fixée au support vasculaire (1) par couture, collage et/ou avec des éléments de serrage, le support vasculaire pouvant présenter dans la zone inférieure une deuxième membrane en forme de coussin.

16. Support vasculaire selon la revendication 15, **caractérisé en ce que** des oeillets, par exemple pour recevoir un ou plusieurs fils, se trouvent sur les arcs, tournés vers l'extérieur, des éléments annulaires situés sur les bords, afin de pouvoir retirer un support vasculaire déjà dilaté dans un cathéter.

17. Support vasculaire selon l'une des revendications précédentes, **caractérisé en ce qu'**il est pourvu, au moins dans des zones partielles, d'un revêtement antiprolifératif ou anticoagulant.
